(19) Europäisches Patentamt
European Patent Office
Office européen des brevets

(11) Numéro de publication : **0 437 128 B1**

(12) # FASCICULE DE BREVET EUROPEEN

(45) Date de publication du fascicule du brevet : **16.02.94 Bulletin 94/07**

(51) Int. Cl.[5] : **A61K 31/495,** A61K 31/535

(21) Numéro de dépôt : **90403501.1**

(22) Date de dépôt : **10.12.90**

(54) **Utilisation des dérivés des fluoroquinolones pour le traitement de la pneumonie à Pneumocystis carinii.**

(30) Priorité : **11.12.89 FR 8916324**

(43) Date de publication de la demande : **17.07.91 Bulletin 91/29**

(45) Mention de la délivrance du brevet : **16.02.94 Bulletin 94/07**

(84) Etats contractants désignés : **AT BE CH DE DK ES FR GB GR IT LI LU NL SE**

(56) Documents cités :
**EP-A- 0 210 513**
**EP-A- 0 219 784**
**EP-A- 0 230 881**
**EP-A- 0 337 231**
**DE-A- 3 632 222**
**DE-A- 3 637 649**
**ANNALS OF INTERNAL MEDICINE, vol. 110, no. 2, 15 janvier 1989, pages 170-171; J. ALTES et al.: "Ciprofloxacin and delirium"**
**THE AMERICAN JOURNAL OF MEDICINE, vol. 87, no. 5A, 30 novembre 1989, pages 119S-120S; L. MENOM et al.: "Brief report: Sequential intravenous/oral ciprofloxacin compared with intravenous ceftazidime in the treatment of serious lower respiratory tract infections"**

(56) Documents cités :
**INFECTION, vol. 17, no. 2, mars-avril 1989, pages 88-89; U. VURMA-RAPP et al.: "Mycobacterium kansasii and pneumocystis carinii pneumonia in a patient with the acquired immunodeficiency syndrome"**
**EUR. J. CANCER CLIN. ONCOL., vol. 25, supplément 2, 1989, pages S53-S61, Pergamon Press plc, GB; J. KLASTERSKY: "Infections in compromised hosts: Considerations on prevention"**
**EUR. J. CANCER CLIN. ONCOL., vol. 24, supplément 1, 1988, pages S15-S24, Pergamon Journals Ltd, GB; D.J. WINSTON et al.: "Prohylaxis of infection in bone marrow transplants"**

(73) Titulaire : **RHONE-POULENC SANTE**
**20, avenue Raymond Aron**
**F-92160 Antony (FR)**

(72) Inventeur : **Gentilini, Marc**
**7 rue de l'Armée Patton**
**F-91640 Briis Sous Forge (FR)**
Inventeur : **Guyot, Anne**
**159 rue Blomet**
**F-75015 Paris (FR)**
Inventeur : **Rosenheim, Michel**
**6 rue des Fontaines du Temple**
**F-75003 Paris (FR)**

(74) Mandataire : **Savina, Jacques et al**
**Rhône-Poulenc Rorer S.A. Direction Brevets (t144) 20 avenue Raymond Aron**
**F-92165 Antony Cédex (FR)**

EP 0 437 128 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne une nouvelle application thérapeutique des dérivés des fluoroquinolones de formule générale :

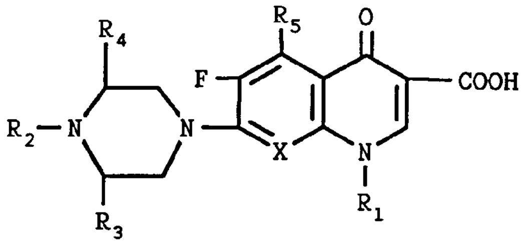

(I)

dans laquelle
$R_1$ est un radical alcoyle contenant 1 à 4 atomes de carbone, fluoroéthyle, cyclopropyle ou difluorophényle,
$R_2$, $R_3$ et $R_4$ sont identiques ou différents et représentent des atomes d'hydrogène ou des radicaux méthyle,
X représente un atome d'azote ou un groupement =CR6- dans lequel $R_6$ est un atome d'hydrogène ou de fluor, ou bien $R_6$ forme avec le radical $R_1$ et les atomes auxquels ils sont rattachés, un hétérocycle à six chaînons substitué par un radical méthyle et contenant éventuellement un atome d'oxygène, et $R_5$ est un atome d'hydrogène, ou peut représenter un radical amino, si $R_6$ est un atome de fluor, ainsi que leurs sels.
Les dérivés des fluoroquinolones qui font l'objet de la présente invention sont largement connus pour leur activité antimicrobienne :
BE 870 576 ; US 4 448 962 ; DE 3 142 854 ; EP 47 005 ; EP 206 283 ; BE 887 574 ; EP 221 463 ; EP 140 116 ; EP 131 839 ; EP 154 780 ; EP 78 362 ; EP 310 849.
Dans Annals of Internal Medicine,110(2), 170-171(1989) a été décrit l'emploi de la ciprofloxacine chez un malade atteint d'affections multiples et traité par ailleurs par le triméthoprime pour une affection à pneumocystis carinii.
Pneumocystis carinii est un microorganisme ubiquitaire capable d'infecter de nombreux mammifères.
Notamment chez l'homme immunodéprimé, il est à l'origine de pneumonies sévères hypoxémiantes et mortelles en l'absence de traitement.
La pneumonie à Pneumocystis carinii survient chez des sujets tels que les nourrissons prématurés ou atteints d'une maladie chronique sévère, les grands enfants ou adultes atteints de cancers, d'hémopathies malignes (leucose, maladie de Hodgkin) soumis à une corticothérapie prolongée, à un traitement immunosuppresseur, ou présentant un syndrome d'immunodéficit acquis (SIDA). La pneumonie à Pneumocystis carinii est en effet l'infection opportuniste du SIDA la plus courante, elle est responsable d'un taux élevé de mortalité chez les malades atteints de cette maladie.

Il a été trouvé, que les fluoroquinolones de formule générale (I) ainsi que leurs sels possèdent une activité anti-pneumocyste particulièrement intéressante, et sont ainsi tout à fait appropriés pour la préparation d'un médicament destiné au traitement préventif et/ou curatif de la pneumocystose de l'homme et/ou de l'animal.
Parmi les produits de formule générale (I), les produits cités ci-après sont les produits préférés : péfloxacine, énoxacine, norfloxacine, ofloxacine, ciprofloxacine, sparfloxacine, léroxacine, loméfloxacine ou témafloxacine.

L'activité a été mise en évidence par le test suivant :

ACTIVITE SUR LA PNEUMOCYSTOSE DU RAT

PREMIERE SERIE D'ESSAIS

On utilise des rats de 200 à 250 g immunodéprimés par deux injections sous-cutanées par semaine de 25 mg d'acétate d'hydrocortisone et un régime pauvre en protéines.
Certains rats reçoivent en outre 10 mg de doxycycline par voie sous-cutanée, deux fois par semaine, de façon à prévenir la survenue d'infections autres que la pneumocystose.
Après deux semaines d'immunodépression l'existence d'une pneumocystose évolutive est vérifiée en sacrifiant des rats et en numérant les pneumocystes présents par gramme de poumon.
Les produits testés pour leur activité anti-pneumocyste sont mis en solution dans du tampon phosphate isotonique à la concentration désirée. Ils sont administrés par voie intra-péritonéale pendant deux semaines.

Deux groupes d'animaux témoins sont constitués :

1/ - rats immunodéprimés, recevant de la doxycycline pendant toute la durée de l'étude (quatre semaines),

2/ - rats immunodéprimés, ne recevant de la doxycycline que pendant les deux premières semaines de l'étude.

Un autre groupe de rats immunodéprimés, recevant de la doxycycline pendant les deux premières semaines de l'étude, reçoit l'association triméthoprime (40 mg/kg) sulfaméthoxazole (200 mg/kg) par voie sous-cutanée deux fois par semaine pendant les deux dernières semaines de l'étude.

Deux groupes d'animaux sont traités par le produit à étudier, il s'agit de rats immunodéprimés, ne recevant de la doxycycline que pendant les deux premières semaines de l'étude puis :

1/ - le produit à étudier 50 mg/kg deux fois par jour par voie intra-péritonéale pendant 14 jours;

2/ - le produit à étudier 100 mg/kg deux fois par jour par voie intra-péritonéale pendant 14 jours.

A la fin des quatre semaines, tous les rats sont sacrifiés, les poumons prélevés et les Pneumocystis carinii sont numérés.

RESULTATS

Les résultats obtenus figurent dans le tableau I ci-après.

- A l'autopsie les poumons des animaux témoins des 1er et 2ème groupes étaient gris brunâtre avec de larges zones oedémateuses. Par contre, ceux des animaux traités par le produit étudié et par l'association triméthoprime-sulfaméthoxazole (3ème groupe témoin) étaient roses et ne montraient aucun signe pathologique. Les poids moyens pulmonaires ne différent pas et sont inférieurs à ceux des animaux témoins.

- Nombre de Pneumocystis carinii :

Les rats témoins des groupes 1 et 2 avaient une moyenne de 3,7 x $10^7$ et 2,6 x $10^7$ pneumocystes par poumon.

Les animaux traités par le produit étudié avaient respectivement une moyenne de 1,3 x $10^5$ pneumocystes (50 mg/kg) et 8,9 x $10^4$ pneumocystes (100 mg/kg) par poumon.

Ces valeurs sont très voisines de celles obtenues à la suite du traitement par l'association triméthoprimesulfaméthoxazole (2,4 x $10^4$ pneumocystes).

CONCLUSION

Le traitement à 50 mg/kg est particulièrement efficace.

DEUXIEME SERIE D'ESSAIS

Dans une autre série d'essais, le groupe témoin est constitué de rats immunodéprimés recevant 10 mg de doxocycline par voie sous-cutanée 2 fois par semaine pendant toute le durée de l'étude (4 semaines).

Un autre groupe de rats immunodéprimés reçoit l'association triméthoprime (40 mg/kg) - sulfaméthoxazole (200 mg/kg), par voie sous-cutanée 2 fois par semaine dès le début de l'immunodépression.

Les produits à étudier sont administrés par voie orale à des rats immunodéprimés, à raison de 100 mg/kg, 3 fois par semaine. On étudie l'action de la péfloxacine, de la témafloxacine et de l'ofloxacine.

Après 4 semaines d'immunodépression, tous les animaux sont sacrifiés et les Pneumocystis carinii intrapulmonaires sont quantifiés.

RESULTATS

Les résultats obtenus figurent dans le tableau II ci-après.

La péfloxacine, la témafloxacine et l'ofloxacine administrés par voie orale, provoquent une diminution du nombre de Pneumocystis carinii.

ACTIVITE SUR LA PNEUMOCYSTOSE MURINE - TABLEAU I

| | 1er groupe témoin + doxycycline (4 semaines) (6 rats) | 2ème groupe témoin + docycycline (2 semaines) (4 rats) | Péfloxacine 50 mg/kg i.p. (5 rats) | Péfloxacine 100 mg/kg i.p. (5 rats) | Groupe recevant triméthoprime-sulfaméthoxazole 40-200 mg/kg s.c. (5 rats) |
|---|---|---|---|---|---|
| Poids initial(g) | 241 ± 17 | 238 ± 15 | 237 ± 12 | 238 ± 12 | 232 ± 8 |
| Poids +4 semaines | 145 ± 16 | 147 ± 9 | 164 ± 6 | 163 ± 6 | 177 ± 10 |
| Rats décédés | 3 | 0 | 0 | 2 | 1 (levure) |
| Rats sacrifiés | 3 | 4 | 5 | 3 | 4 |
| Poids poumon (g) | 1,44 ± 0,31 | 1,36 ± 0,28 | 0,95 ± 0,04 | 0,86 ± 0,04 | 1,00 ± 0,15 |
| Nombre de pneumocystes/g de poumon | $3x10^7 \pm 1,7x\ 10^7$ | $1,7x10^7 \pm 2,4x10^7$ | $1,4x10^5 \pm 1x10^5$ | $9,8x10^4 \pm 12x10^4$ | $2,3x10^4 \pm 2,9x10^4$ |
| Nombre de pneumocystes par poumon | $3,7x10^7 \pm 2,1x10^7$ | $2,6x10^7 \pm 2,8x10^7$ | $1,3x10^5 \pm 1x10^5$ | $8,9x10^4 \pm 10x10^4$ | $2,4x10^4 \pm 2,8x10^4$ |

Après les deux premières semaines d'immunodépression 3 rats ont été sacrifiés : nombre de pneumocystes = $2x10^6$/g de poumon.

ACTIVITE SUR LA PNEUMOCYSTOSE MURINE - TABLEAU II

| | groupe témoin + doxycycline (4 semaines) (15 rats) | Péfloxacine 100 mg/kg p.o. (5 rats) | Témafloxacine 100 mg/kg p.o. (5 rats) | Ofloxacine 100 mg/kg p.o. (5 rats) | Groupe recevant triméthoprime-sulfaméthoxazole 40-200 mg/kg s.c. (10 rats) |
|---|---|---|---|---|---|
| Poids initial(g) | 223 ± 18 | 237 ± 13 | 210 ± 6 | 236 ± 9 | 237 ± 14 |
| Poids +4 semaines | 164 ± 22 | 183 ± 18 | 160 ± 12 | 205 ± 25 | 178 ± 11 |
| Rats décédés | 2 | 0 | 0 | 0 | 1 |
| Rats sacrifiés | 13 | 5 | 5 | 5 | 9 |
| Nombre de pneumocystes/g de poumon | $4,8x10^6$<br>$2,1x10^6$ - $1,1x10^7$ | $8,0x10^3$<br>$3,0x10^2$ - $2,1x10^5$ | $6,1x10^5$<br>$2,1x10^5$ - $1,8x10^6$ | $7,9x10^5$<br>$1,4x10^5$ - $4,4x10^6$ | $2,0x10^3$<br>$1,0x10^3$ - $3,3x10^3$ |

La présente invention concerne l'obtention d'un médicament contenant au moins un produit de formule générale (I) éventuellement sous forme de sel, à l'état pur ou sous forme d'une composition pharmaceutique en association avec un ou plusieurs diluants ou adjuvants compatibles.

Ces compositions peuvent être utilisées par voie orale, parentérale ou en aérosols.

Les compositions peuvent être utilisées à titre curatif ou à titre préventif chez des sujets présentant une immunodéficience et/ou infectés par Pneumocystis carinii et/ou présentant un risque de contamination par Pneumocystis carinii. Bien entendu, la constitution de ces compositions sera adaptée au cas particulier du tractus digestif des immunodéprimés.

Comme compositions solides pour administration orale peuvent être utilisés des comprimés, des pilules, des gélules, des poudres ou granulés. Dans ces compositions, le produit actif selon l'invention est mélangé à un ou plusieurs diluants ou adjuvants inertes, tels que saccharose, lactose ou amidon. Ces compositions peuvent comprendre des substances autres que les diluants, par exemple un lubrifiant tel que le stéarate de magnésium.

Comme compositions liquides pour administration orale, on peut utiliser des solutions pharmaceutiquement acceptables, des suspensions, des émulsions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale, peuvent être des solutions stériles aqueuses ou non aqueuses, des suspensions ou des émulsions. Comme solvant ou véhicule, on peut employer le propylèneglycol, un polyéthylèneglycol, des huiles végétales, en particulier l'huile d'olive, des esters organiques injectables, par exemple l'oléate d'éthyle. Ces compositions peuvent également contenir des adjuvants, en particulier des agents mouillants, isotonisants, émulsifiants, dispersants et stabilisants. La stérilisation peut se faire de plusieurs façons, par exemple à l'aide d'un filtre bactériologique, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions destinées à l'usage sous forme d'aérosols liquides peuvent être des solutions stériles stables ou de compositions solides dissoutes au moment de l'emploi dans de l'eau stérile apyrogène, du sérum ou tout autre véhicule pharmaceutiquement acceptable.

Les aérosols peuvent être également des aérosols secs destinés à être directement inhalés, dans lesquels le principe actif finement divisé est associé à un diluant ou véhicule solide hydrosoluble d'une granulométrie de 30 à 80 microns, comme par exemple le dextrane, le mannitol ou le lactose.

En thérapeutique humaine, le médecin déterminera la posologie qu'il estime la plus appropriée en fonction d'un traitement préventif ou curatif, en fonction de l'âge, du poids, du degré de l'infection et des autres facteurs propres au sujet à traiter. Généralement, les doses sont comprises entre 1 et 5 g par jour par voie orale pour un adulte.

Les exemples suivants illustrent des compositions selon l'invention destinées au traitement de la pneumocystose :

## EXEMPLE A

On prépare des comprimés dosés à 400 mg ayant la composition suivante :
Péfloxacine sous forme de mésylate dihydraté : 400 mg
Excipient :
**noyau** : amidon de blé, gélatine, talc, stéarate de magnésium, carboxyméthylamidon sodique q.s.p. un noyau;
**enrobage** : hydroxypropylméthylcellulose, éthylcellulose, sébacate de dibutyle, oxyde de titane, talc, polyoxyéthylène glycol 6000.

## EXEMPLE B

On prépare des comprimés dosés à 800 mg ayant la composition suivante :
Péfloxacine sous forme de mésylate dihydraté : 800 mg
Excipient :
**noyau** : amidon de blé, gélatine, talc, stéarate de magnésium, carboxyméthylamidon sodique q.s.p. un noyau;
**enrobage** : hydroxypropylméthylcellulose, éthylcellulose, sébacate de dibutyle, oxyde de titane, talc, polyoxyéthylène glycol 6000.

Un autre objet de la présente invention concerne aussi les compositions à usage vétérinaire préventif ou curatif contre la pneumocystose.

Les compositions à usage vétérinaire pourront être utilisées sous les différentes formes galéniques injectables précédemment décrites pour l'administration chez l'homme. Elles peuvent être aussi des aliments pour animaux ou des mélanges concentrés destinés à l'alimentation animale, renfermant une quantité suffisante du dérivé de formule générale (I). Plus précisément, elles peuvent se présenter sous forme de poudres hydrosolubles à mélanger à l'alimentation.

D'une manière générale, on emploiera la posologie la plus appropriée en fonction du poids de l'animal, du degré

de l'infection et des autres facteurs propres pouvant intervenir, étant entendu que la dose convenable pour produire un effet peut varier dans d'assez larges limites.

## Revendications

1. Application d'un dérivé des fluoroquinolones de formule générale :

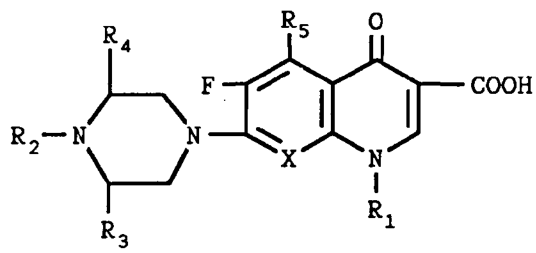

dans laquelle
$R_1$ est un radical alcoyle contenant 1 à 4 atomes de carbone, fluoroéthyle, cyclopropyle ou difluorophényle, $R_2$, $R_3$ et $R_4$ sont identiques ou différents et représentent des atomes d'hydrogène ou des radicaux méthyle, X représente un atome d'azote ou un groupement $=CR_6$- dans lequel $R_6$ est un atome d'hydrogène ou de fluor, ou bien $R_6$ forme avec le radical $R_1$ et les atomes auxquels ils sont rattachés, un hétérocycle à six chaînons substitué par un radical méthyle et contenant éventuellement un atome d'oxygène, et $R_5$ est un atome d'hydrogène, ou peut représenter un radical amino si $R_6$ est un atome de fluor, ainsi que de ses sels, pour obtenir un médicament destiné au traitement préventif et/ou curatif de la pneumocystose.

## Claims

1. Application of a fluoroquinolone derivative of general formula:

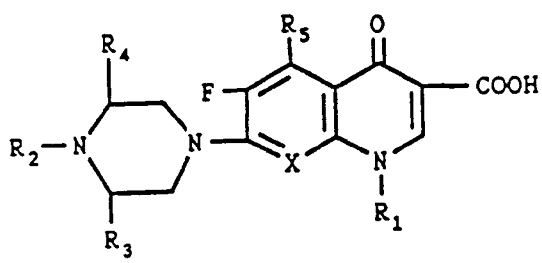

in which
$R_1$ is an alkyl radical containing 1 to 4 carbon atoms or a fluoroethyl, cyclopropyl or difluorophenyl radical, $R_2$, $R_3$ and $R_4$ are identical or different and represent hydrogen atoms or methyl radicals, X represents a nitrogen atom or a group $=CR_6$- in which $R_6$ is a hydrogen or fluorine atom, or alternatively $R_6$ with the radical $R_1$ and the atoms to which they are attached forms a six-membered heterocycle substituted with a methyl radical and optionally containing an oxygen atom, and $R_5$ is a hydrogen atom, or can represent an amino radical if $R_6$ is a fluorine atom, as well as its salts, for obtaining a medicinal product intended for the preventive and/or curative treatment of pneumocystosis.

## Patentansprüche

1. Anwendung eines Derivats der Fluorchinolone der allgemeinen Formel

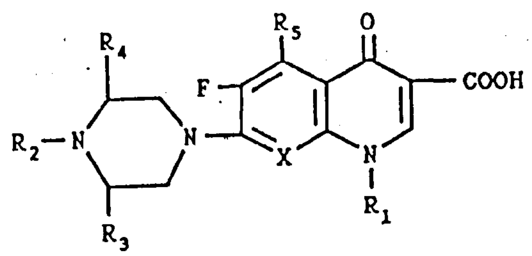

worin

$R_1$ ein Alkylrest mit 1 bis 4 Kohlenstoffatomen, Fluorethyl, Cyclopropyl oder Difluorphenyl ist, $R_2$, $R_3$ und $R_4$ identisch oder verschieden sind und Wasserstoffatome oder Methylreste bedeuten, X ein Stickstoffatom bedeutet oder eine Gruppe $=CR_6-$ worin $R_6$ ein Wasserstoff- oder Fluoratom ist, oder $R_6$ bildet auch mit dem Rest $R_1$ und den Atomen, an das sie gebunden sind, einen Heterozyklus mit 6 Kettengliedern, substituiert durch einen Methylrest und enthaltend gegebenenfalls ein Sauerstoffatom und $R_6$ ist ein Wasserstoffatom oder kann einen Aminorest darstellen, wenn $R_6$ ein Fluoratom ist, sowie seine Salze, zur Erzielung eines Arzneimittels, das zur vorbeugenden oder heilenden Behandlung der Pneumocystose bestimmt ist.